# EUROPÄISCHE PATENTSCHRIFT

(11) **EP 1 560 518 B1**
(45) Veröffentlichungstag und Bekanntmachung des Hinweises auf die Patenterteilung: **06.09.2006**
(21) Anmeldenummer: 03810986.4
(22) Anmeldetag: 13.11.2003
(51) Int. Cl.: A61B 6/00

(54) **DECKENSTATIV ZUR AUFNAME EINER MEDIZINTECHNISCHEN STRAHLENQUELLE**
CEILING SUPPORT FOR A MEDICO-TECHNICAL RADIATION SOURCE
SUPPORT PLAFONNIER DESTINE A RECEVOIR UNE SOURCE DE RAYONNEMENT MEDICO-TECHNIQUE

(30) Priorität: 14.11.2002 DE 10252931
(43) Veröffentlichungstag der Anmeldung: 10.08.2005
(73) Patentinhaber: Hans Pausch GmbH & Co., 91056 Erlangen (DE)
(72) Erfinder: STEGER, Rainer, 91353 Hausen (DE); BRILL, Michael, 91056 Erlangen (DE)
(74) Vertreter: Gassner, Wolfgang
(86) Internationale Anmeldenummer: PCT/EP2003/012667
(87) Internationale Veröffentlichungsnummer: WO 2004/043261

(56) Entgegenhaltungen:
- US-A- 2 876 362

## Beschreibung

Die Erfindung betrifft ein Deckenstativ zur Aufnahme einer medizintechnischen Strahlenquelle.

Ein derartiges Deckenstativ ist beispielsweise aus der US 3,175,085 bekannt.

Zum Ausgleich des Gewichts einer am freien Ende des Teleskops bzw. Teleskoparms angebrachten Strahlenquelle ist üblicherweise ein Seil durch den Teleskoparm geführt, das mit einer Einrichtung zur Kompensation des Gewichts verbunden ist. Eine solche Einrichtung kann beispielsweise eine elektromotorisch angetriebene Seilwinde umfassen. Damit lässt sich die Strahlenquelle allerdings nicht mit der Dynamik eines manuell verstellbaren Deckenstativs bewegen.

Zur Kompensation des Gewichts sind ferner Einrichtungen bekannt, die eine Gasdruck- oder Torsionsfeder umfassen..Solche Einrichtungen haben den Nachteil, dass die Gewichtskompensation nicht über die gesamte Auszugslänge des Teleskoparms konstant ist. Torsionsfedern haben den weiteren wesentlichen Nachteil einer relativ geringen Lebensdauer.

Des Weiteren ist es bekannt, zur Gewichtskompensation eine Zugfeder zu verwenden und das Seil über die Spiralwinde zu führen. Damit kann zwar über einen weiten Abschnitt der Auszugslänge des Teleskoparms annähernd eine konstante Kompensation des Gewichts erreicht werden. Ein Deckenstativ unter Verwendung von Zugfedern kann aber nicht kompakt ausgestaltet werden. Außerdem müssen zusätzliche sicherheitstechnische Einrichtungen für den Fall eines eventuellen Bruchs der Zugfedern vorgesehen werden..

Aufgabe der Erfindung ist es, ein Deckenstativ zur Aufnahme einer medizintechnischen Strahlenquelle anzugeben, das möglichst einfach aufgebaut, langlebig und in einem weiten Bereich auf das jeweilige Gewicht der Strahlenquelle einstellbar ist.

Diese Aufgabe wird durch die Merkmale des Anspruchs 1 gelöst. Zweckmäßige Ausgestaltungen ergeben sich aus den Merkmalen der Ansprüche 2 bis 14.

Nach Maßgabe der Erfindung ist ein Deckenstativ zur Aufnahme einer medizintechnischen Strahlenquelle vorgesehen, mit einem aus mehreren ineinander fahrbaren Holmen gebildeten Teleskop, das über ein erstes Seil mit einer Einrichtung zur Kompensation des Gewichts einer am freien Ende des Teleskops anzubringenden Strahlenquelle verbunden ist, wobei die Einrichtung zur Kompensation umfasst:
eine Seiltrommel zum Auf- und Abrollen des ersten Seils,
eine fest mit der Seiltrommel verbundene Spiralwinde,
wobei die Spiralwinde mittels eines zweiten Seils mit einem gegen die Kraft einer Druckfeder verschiebbaren Gleitelement verbunden ist, und
wobei ein Radius der Spiralwinde mit zunehmender Druckkraft der Druckfeder abnimmt, so dass ein das Gewicht kompensierendes Moment unabhängig von der jeweiligen Länge des Tele'skops im Wesentlichen gleich bleibt.

Der Begriff "Seil" ist im Zusammenhang mit der vorliegenden Erfindung allgemein im Sinne eines aufwickelbaren Verbindungsmittels zu verstehen. Insoweit fallen unter den Begriff "Seil" auch Verbindungsmittel wie z. B. eine Kette, ein Metall- oder Gewebeband, ein Kunststoffriemen, ein Kunststoffseil und dgl.. Die "Seiltrommel" sowie die "Spiralwinde" sind an ihrem Außenumfang jeweils so gestaltet, dass damit das jeweils verwendete "Seil" aufgewickelt werden kann.

Das vorgeschlagene Deckenstativ ist einfach aufgebaut. Die vorgeschlagene Verwendung einer Druckfeder trägt zu einer wesentlich verbesserten Haltbarkeit und Betriebssicherheit bei. Außerdem wird damit eine Einstellung des zu kompensierenden Gewichts in einem weiten Bereich ermöglicht.

Vorzugsweise ist die Spiralwinde fest an der Seiltrommel angebracht, d.h. sie ist gegenüber der Seiltrommel nicht verdrehbar. In diesem Fall können das erste und das zweite Seil auch durch ein einziges Seil ersetzt sein, welche am einen Ende mit dem Teleskop und am anderen Ende mit dem verschiebbaren Gleitelement verbunden ist. In diesem Fall ist das Seil allerdings am Ende der Seiltrommel und/oder am Ende der Spiralwinde befestigt.

Nach einer vorteilhaften Ausgestaltung ist die Abnahme des Radius nicht linear. Sie kann ähnlich einer Hyperbel verlaufen. Damit kann über den gesamten Auszugsbereich des Teleskoparms eine im Wesentlichen gleich bleibende Kompensation des Gewichts der Strahlenquelle erreicht werden. Zweckmäßigerweise ist die Spiralwinde in Form einer hyperbolischen Spiralwinde ausgebildet. Mit einer hyperbolischen Ausbildung der Spiralwinde wird in der Praxis eine gleich bleibende Gewichtskompensation erreicht.

Nach einer weiteren Ausgestaltung sind zwei Druckfedern vorgesehen, die auf parallel zueinander verlaufenden Führungsrohren angeordnet sind. Die vorgeschlagene Anordnung der Druckfedern auf Führungsrohren trägt zu einer verbesserten Knicksicherheit bei. Selbst bei einem Bruch einer der Druckfedern kommt es nicht zu einem plötzlichen Absacken der Strahlenquelle und damit zu einer Gefährdung von Personen.

Vorteilhafterweise ist das Gleitelement ein gegen die Kraft der Druckfedern auf den Führungsrohren verschiebbares Querhaupt. Das zweite Seil ist zweckmäßigerweise um eine am Querhaupt angebrachte Rolle geführt, mit seinem einen Ende an einem den Teleskoparm aufnehmenden Rahmen und an seinem anderen Ende am äußeren Radius der Spiralwinde befestigt. Das eine Ende des zweiten Seils kann auch an einer Außenwand des Teleskoparms angebracht sein. Die von den Druckfedern über das Querhaupt auf die Spiralwinde und damit auf die Seiltrommel ausgeübte Kompensationskraft wird durch das Vorsehen der als lose Rolle wirkenden Rolle nach dem Prinzip des Flaschenzugs halbiert.

Von Vorteil ist es weiter, dass die Spiralwinde zwischen den beiden Druckfedern angebracht ist. Es kann damit eine besonders'kompakte Bauweise erzielt werden.

Zur Erhöhung der Betriebssicherheit sind zweckmäßigerweise zwei erste und zwei zweite Seile vorgesehen. Selbst bei einem Bruch eines der Seile bleibt das Deckenstativ uneingeschränkt betriebsfähig. Aus sicherheitstechnischen Erwägungen kann die Spiralwinde des Weiteren mit einer Permanentmagnetbremse verbunden sein, derart, dass bei einem Stromausfall die Spiralwinde gebremst wird. Eine solche Bremse wird üblicherweise nur beim vertikalen Verstellen des Deckenstativs gelöst. Eine am Deckenstativ aufgenommene Strahlenquelle kann damit zusätzlich gegen eine versehentlich ausgelöste Vertikalbewegung gesichert werden.

Nach einer weiteren Ausgestaltung ist eine Vorrichtung zum Einstellen einer auf das Gleitelement wirkenden Vorspannung der Druckfeder/n vorgesehen. Damit ist es möglich, einen Bereich einzustellen, in dem die Druckfedern eine annähernd lineare Kennlinie aufweisen. Des Weiteren kann eine Einrichtung zur stufenlosen Einstellung der Federrate vorgesehen sein, die zweckmäßigerweise als eine die Druckfeder/n gegen das Führungsrohr verspannende Klemmmanschette/n ausgebildet ist. Damit kann ein weiter Gewichtsbereich kompensiert werden.

Nach einer besonders bevorzugten Ausgestaltung sind an einer Innenseite mindestens einer der Holme mehrere-axial verlaufende hinterschnittene Nuten vorgesehen, und im Wesentlichen stegförmig ausgebildete Führungsschienen sind mittels einer Schraubverbindung mit in den Nuten aufgenommenen Nutensteinen befestigt. Zweckmäßigerweise sind an der Innenseite der Holme gleichmäßig über deren Umfang drei Nuten vorgesehen. Die vorgeschlagene Anbringung der Führungsschienen an der Innenseite der Holme erleichtert wesentlich die Montage des Teleskoparms. Die Führungsschienen können bei der Montage der Holme in einem gewissen Bereich radial verschoben und erst dann mit den Holmen verspannt werden.

Nachfolgend wird ein Ausführungsbeispiel der Erfindung anhand der Zeichnung näher erläutert. Es zeigen:
- Fig. 1: eine erste perspektivische Ansicht des Deckenstativs,
- Fig. 2: eine zweite perspektivische Ansicht des Deckenstativs nach Fig. 1,
- Fig. 3: eine perspektivische Ansicht der Spiralwinde nach Fig. 2,
- Fig. 4: eine perspektivische Ansicht einer Spiralwinde nach Fig. 3 mit elekromptorischem Antrieb,

- Fig. 5: eine aufgebrochene perspektivische Ansicht des Dekkenstativs,
- Fig. 5a: eine Detailansicht gemäß dem Ausschnitt A in Fig. 5 und
- Fig. 6: eine Querschnittsansicht durch den Teleskoparm.

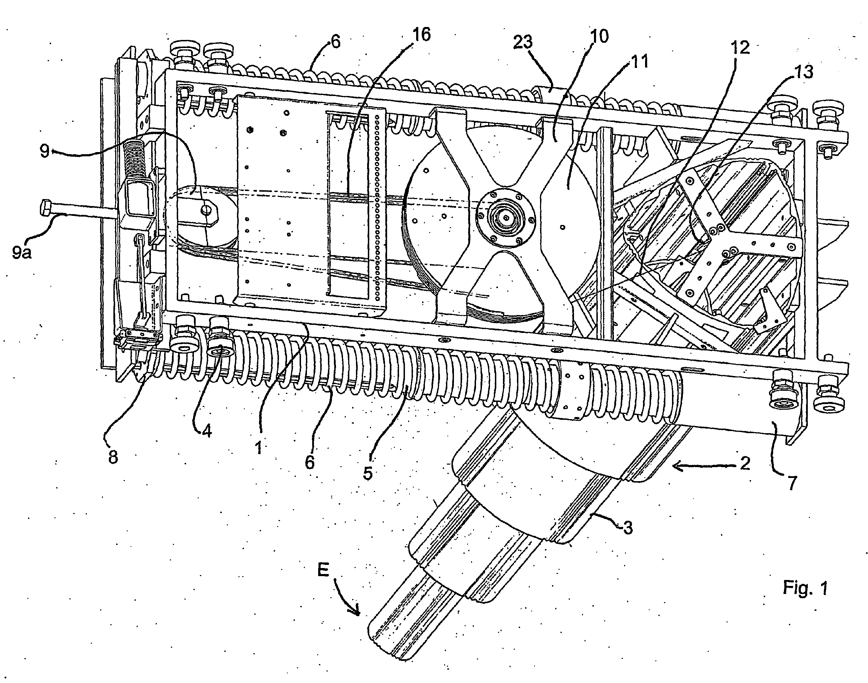

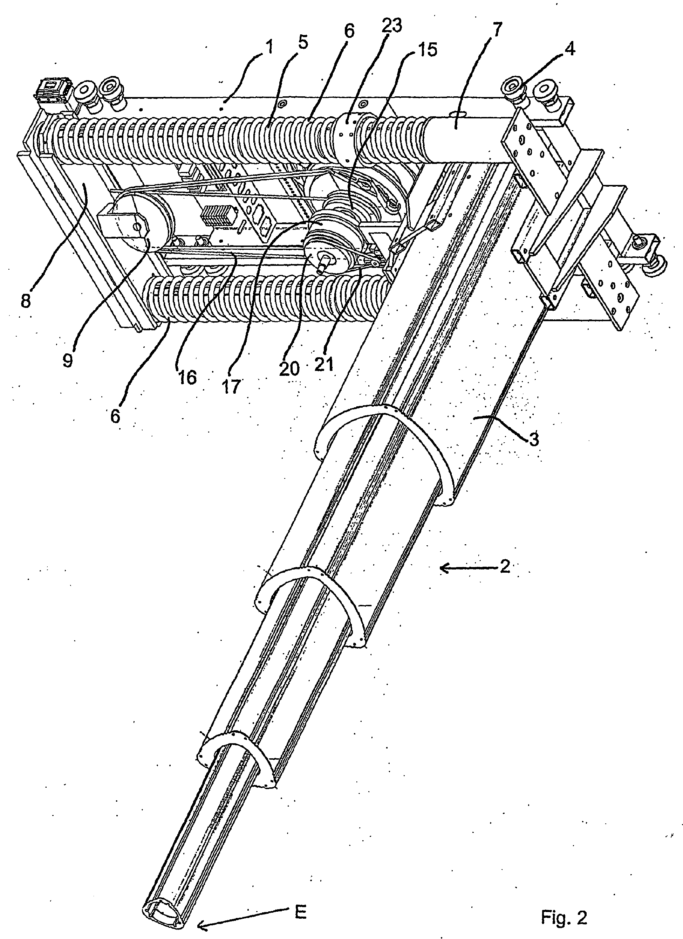

In den Fig. 1 und 2 ist perspektivisch ein erfindungsgemäßes Deckenstativ gezeigt. An einem rechteckigen Rahmen 1 ist ein etwa senkrecht davon sich erstreckender Teleskoparm 2 angebracht, der mehrere ineinander fahrbare Holme 3 aufweist. Der Rahmen 1 ist mit Laufrollen 4 versehen, mit denen er auf an einer Decke eines Raums abgebrachten Schienen (hier nicht gezeigt) verfahrbar sein kann. Die Holme 3 sind zweckmäßigerweise aus Aluminiumstrangpressprofileri. An den einander gegenüberliegenden Längsseiten des Rahmens 1 sind Führungsrohre 5 angebracht, auf denen jeweils mindestens eine - im vorliegenden Ausführungsbeispiel zwei aneinander liegende - Druckfedern 6 aufgenommen sind. Das eine Ende der Druckfedern 6 ist gegen eine auf jedem der Führungsrohre 5 aufgenommene Hülse 7 und das andere Ende gegen ein auf den Führungsrohren 5 aufgenommenes Querhaupt 8 abgestützt. An der dem Teleskoparm 2 zugewandten Seite des Querhaupts 8 ist mittig eine erste als lose Rolle wirkende Umlenkrolle 9 angebracht. Der Abstand des Querhaupts 8 zur Achse der Seiltrommel 11 kann mittels einer Stellschraube 9a verändert werden. Mit der Stellschraube 9a lässt sich also die auf das Querhaupt 8 wirkende Verspannung einstellen. Etwa in der Mitte des Rahmens 1 ist ein Quertragelement 10 befestigt, an dem drehbar eine Seiltrommel 11 aufgenommen ist. Die Seiltrommel 11 weist an ihrem Außenumfang Führungsrillen zur Aufnahme eines ersten Seils 12 auf. Das in Fig. 1 schematisch angedeutete erste Seil 12 ist über eine zweite feste Umlenkrolle 13 geführt und mit dem das freie Ende E des Teleskoparms 2 bildenden Holm 3 fest verbunden (hier nicht gezeigt). An der dem Quertragelement 10 abgewandten Seite ist unter Zwischenschaltung einer Distanzscheibe 14 eine hyperbolische Spiralwinde 15 fest mit der Seiltrommel 11 verbunden. Die Spiralwinde 15 weist an ihrem Außenumfang Führungsrillen zur Aufnahme eines zweiten - im vorliegenden Ausführungsbeispiel zwei zweiter - Seile 16 auf. Wegen der Definition einer hyperbolischen Spirale wird beispielhaft verwiesen auf BRONSTEIN-SEMENDJAJEW, Taschenbuch der Mathematik, 18. Auflage, 1979, Seite 92.

Die zweiten Seile 16 sind, wie aus Fig. 2 erkennbar ist, mit ihrem einen Ende in der Nähe des maximalen Radius der Spiralwinde 151 und mit ihrem anderen Ende an der Außenwand des am Rahmen 1 angebrachten Holms 3 befestigt (hier nicht gezeigt). Die zweiten Seile 16 sind außerdem um die erste Umlenkrolle 9 geführt.

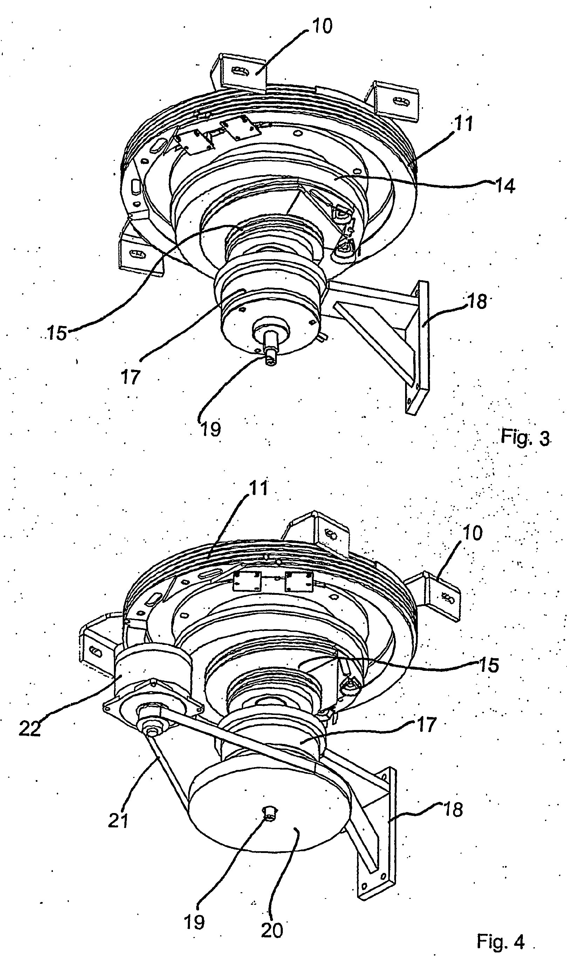

Mit der Spiralwinde 15 fest verbunden ist eine Permanentmagnetbremse 17, die mittels einer Halterung 18 fest am Rahmen 1 angebracht ist. Eine sich von der Permanentmagnetbremse 17 erstreckende Achse 19 kann optional (siehe Fig. 2 und 4) mit einer Riemenscheibe 20 versehen sein, die über einen Keilriemen 21 mit einem Elektromotor 22 antriebsmäßig verbunden ist.

Zur Einstellung der Federrate der Druckfedern 6 sind Klemmelemente 23 vorgesehen, mit denen die Druckfedern 6 gegen die Führungsrohre 5 verspannbar sind. Damit kann die Länge der Druckfedern 6 stufenlos eingestellt und damit der gewünschte zu kompensierende Gewichtsbereich gewählt werden.

In den Fig. 1 und 2 ist das Deckenstativ der Übersichtlichkeit halber mit voll ausgezogenem Teleskoparm 2 und gleichzeitig mit nicht komprimierten Druckfedern 6 gezeigt. Der funktionelle Zusammenhang der bewegbaren Komponenten des Dekkenstativs ist darin nicht wiedergegeben.

Die Funktion des. Deckenstativs ist wie folgt:

Eine am freien Ende E angebrachte Strahlenquelle (hier nicht gezeigt) übt auf den Teleskoparm 2 eine Gewichtskraft aus. Die Gewichtskraft wird über das erste Seil 12 und die zweite Umlenkrolle 13 auf die Seiltrommel 11 übertragen. Auf die Seiltrommel 11 und die damit fest verbundene Spiralwinde 15 wirkt ein der Gewichtskraft entsprechendes konstantes Drehmoment. Dieses konstante Drehmoment wird durch ein auf die Spiralwinde 15 ausgeübtes entgegengesetztes Drehmoment kompensiert. Das entgegengesetzte Drehmoment wird durch die auf das Querhaupt 8 wirkende Druckkraft der Druckfedern 6 erzeugt, welche durch die zweiten Seile 16 übertragen wird. Die mit zunehmender Kompression der Druckfedern 6 zunehmende Druckkraft wird durch eine nicht lineare Verkleinerung des Radius der Spiralwinde 15 so kompensiert, dass das entgegengesetzte Drehmoment über die gesamte Auszugslänge des Teleskoparms 2 konstant ist.

Im vollständig ausgezogenen Zustand des Teleskoparms 2 sind die Druckfedern 6 maximal komprimiert. Das Querhaupt 8 ist um einen maximalen Betrag in Richtung des Teleskoparms 2 verschoben. Die zweiten Seile 16 liegen in diesem Fall am minimalen Radius der Spiralwinde 15 an. Beim Aufwärtsbewegen des Teleskoparms 2 dreht sich die Spiralwinde 15 so, dass die zweiten Seile 16 von einem sich ständig vergrößernden Radius aufgewickelt werden. Pro Drehwinkeleinheit der Spiralwinde 15 wird beim Aufwickeln also stetig ein größerer Längenabschnitt der zweiten Seile aufgewickelt. Damit einher geht die gleichzeitig abnehmende Druckkraft der Druckfedern 6, so dass das entgegengesetzte Drehmoment konstant gehalten'wird.

Zum Bremsen einer Verschiebebewegung des Teleskoparms 2 kann optional die Permanentmagnetbremse 17 vorgesehen sein. Sie ist zweckmäßigerweise so ausgestaltet, dass bei unterbrochenem Stromkreis die Permanentmagnetbremse 17 die Spiralwinde 15 bremst. So kann es im Falle eines Stromausfalls nicht zu einer unerwünschten Verschiebebewegung des Teleskoparms 2 kommen.

Die Spiralwinde 15 kann ferner über die Achse 19 mit einem elektromotorischen Antrieb 20, 21, 22 verbunden sein. Ein solcher elektromotorischer Antrieb dient der Unterstützung der Verschiebebewegung des Teleskoparms 2.

Die zu kompensierende Gewichtskraft kann durch Verstellen der Klemmbacken 23 stufenlos innerhalb eines weiten Bereichs eingestellt werden. Dadurch ist es möglich, die wirksame Länge der Druckfedern 6 zu ändern.

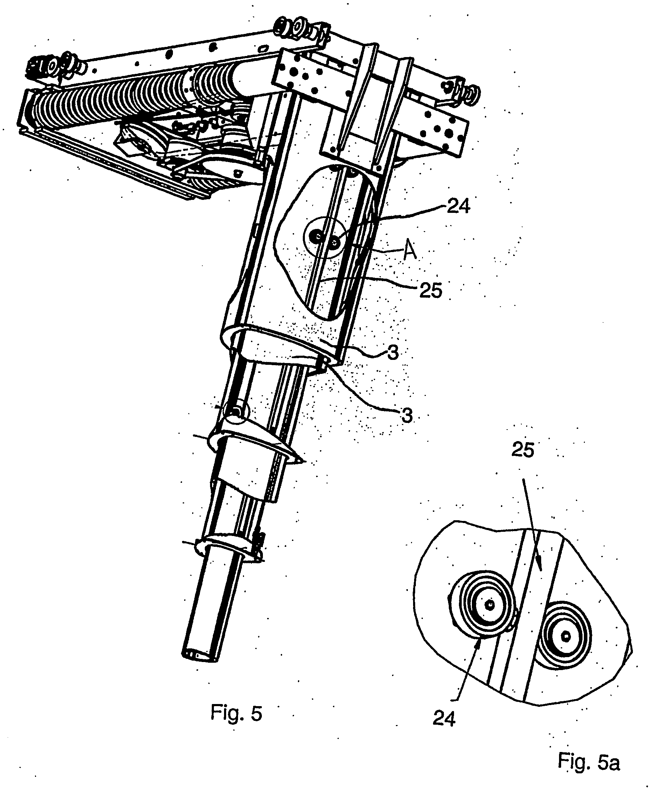

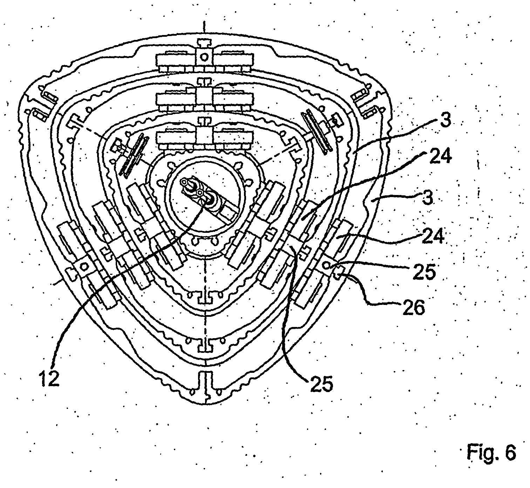

In den Fig. 5, 5a und 6 ist eine besonders bevorzugte Ausführung der Führung der Holme 3 gezeigt. Die nachfolgend beschriebene besonders bevorzugte Ausführung der Holme 3 kann auch für sich genommen eine unabhängige Erfindung darstellen. Bei der Montage des Teleskoparms 2 tritt in der Praxis das Problem auf, dass an der Innenseite der Holme vorgesehene Führungsschienen 25 zum Ausgleich von Fertigungstoleranzen der Holme 3 exakt justiert werden müssen. Anderenfalls kommt es beim Ausziehen des Teleskoparms 2 zum Abheben von auf den Führungsschienen 25 laufenden Rollen 24. Um eine ausreichende Vorspannung der'Rollen 24 gegen die Flanken der Führungsschienen 25 zu gewährleisten, sind die Rollen 24 exzentrisch befestigt. Sie können bei der Montage gegen die Führungsschienen 25 vorgespannt werden. Nach dem bevorzugten Ausführungsbeispiel sind in den Holmen 3 axial verlaufende hinterschnittene Nuten 26 vorgesehen. Bei einem hier gezeigten im Wesentlichen dreieckigen Profil der Holme 3 verlaufen die Nuten 26 an der Innenseite der Holme 3 etwa mittig und sind um 120° versetzt. In die Nuten 26 werden zur Befestigung der Führungsschienen 25 mit einem Gewinde versehene Nutensteine (hier nicht gezeigt) eingefädelt. Die Führungsschienen 25 werden anschließend mittels einer Schraube mit den Nutensteinen verbunden. Zur Montage werden anschließend zwei Holme 3 ineinander geschoben, wobei die Führungsschienen 25 jeweils noch verschiebbar an der Innenseite des äußeren Holms 3 befestigt sind. Die Rollen 24 werden gegen die Führungsschienen 25 in Anlage gebracht. Sodann wird der innere Holm 3 im äußeren Holm verfahren. Infolgedessen justieren sich die Führungsschienen 25 selbsttätig. Sie müssen anschließend lediglich noch fest gezogen werden.

### Bezugszeichenliste

- 1: Rahmen
- 2: Teleskoparm
- 3: Holm
- 4: Laufrolle
- 5: Führungsrohr
- 6: Druckfeder
- 7: Hülse
- 8: Querhaupt
- 9: erste Umlenkrolle
- 10: Quertragelement
- 11: Seiltrommel
- 12: erstes Seil
- 13: zweite Umlenkrolle
- 14: Distanzscheibe
- 15: Spiralwinde
- 16: zweites Seil
- 17: Permanentmagnetbremse
- 18: Halteelement
- 19: Achse
- 20: Riemenscheibe
- 21: Keilriemen
- 22: Elektromotor
- 23: Klemmbacken
- 24: Rolle
- 25: Führungsschiene
- 26: Nut

- E: freies Ende

## Patentansprüche

1. Deckenstativ zur Aufnahme einer medizintechnischen Strahlenquelle,
mit einem aus mehreren ineinander fahrbaren Holmen (3) gebildeten Teleskop (2),
das über ein erstes Seil (12) mit einer Einrichtung zur Kompensation des Gewichts der am freien Ende des Teleskops (2) anzubringenden Strahlenquelle verbunden ist,
wobei die Einrichtung zur Kompensation umfasst:
eine Seiltrommel (11) zum Auf- und Abrollen des ersten Seils (12),
eine mit der Seiltrommel, (11) verbundene Spiralwinde (15),
wobei die Spiralwinde (15) mittels eines zweiten Seils (16) mit einem gegen die Kraft einer Druckfeder (6) verschiebbaren Gleitelement (8) verbunden ist, und
wobei ein Radius der Spiralwinde (15) mit zunehmender Druckkraft der Druckfeder (6) abnimmt, so dass ein das Gewicht kompensierendes Moment unabhängig von der jeweiligen Länge des Teleskops (2) im Wesentlichen gleich bleibt.

2. Deckenstativ nach Anspruch 1, wobei die Spiralwinde (15) fest an der Seiltrommel (11) angebracht ist.

3. Deckenstativ nach Anspruch 1 oder 2, wobei die Abnahme des Radius nicht linear ist.

4. Deckenstativ nach einem der vorhergehenden Ansprüche, wobei die Spiralwinde (15) eine hyperbolische Spiralwinde ist.

5. Deckenstativ nach einem der vorhergehenden Ansprüche, wobei zwei Druckfedern (6) vorgesehen sind, die auf parallel zueinander verlaufenden Führungsrohren (5) angeordnet sind.

6. Deckenstativ nach einem der vorhergehenden Ansprüche, wobei das Gleitelement ein gegen die Kraft der Druckfedern (6) auf den Führungsrohren (5) verschiebbares Querhaupt (8) ist.

7. Deckenstativ nach einem der vorhergehenden Ansprüche, wobei das zweite Seil (16) um eine am Querhaupt (8) angebrachte Rolle (9) geführt, mit seinem einen Ende an einem das Teleskop (2) aufnehmenden Rahmen (1) und mit seinem anderen Ende beim maximalen Radius der Spiralwinde (15) befestigt ist.

8. Deckenstativ nach einem der vorhergehenden Ansprüche, wobei die Spiralwinde (15) zwischen den beiden Druckfedern (6) angebracht ist.

9. Deckenstativ nach einem der vorhergehenden Ansprüche, wobei zwei erste (12) und zwei zweite Seile (16) vorgesehen sind.

10. Deckenstativ nach einem der vorhergehenden Ansprüche, wobei die Spiralwinde (15) mit einer Permanentmagnetbremse (17) verbunden ist, derart, dass bei einem Stromausfall die Spiralwinde (15) gebremst wird.

11. Deckenstativ nach einem der vorhergehenden Ansprüche, wobei die Spiralwinde (15) mit einen elektromotorischen Antrieb (20, 21, 22) verbunden ist.

12. Deckenstativ nach einem der vorhergehenden Ansprüche, wobei eine Vorrichtung (23) zur Einstellung einer auf das Gleitelement (8) wirkenden Vorspannung der Druckfeder/n (6) vorgesehen ist.

13. Deckenstativ nach einem der vorhergehenden Ansprüche, wobei eine Vorrichtung zur stufenlose Einstellung der Federrate vorgesehen ist, die vorzugsweise als eine die Druckfeder (6) gegen das Führungsrohr (5) verspannende Klemmmanschette (23) ist.

14. Deckenstativ nach einem der vorhergehenden Ansprüche, wobei an einer Innenseite mindestens eines der Holme (3) mehrere axial verlaufende hinterschnittene Nuten (26) vorgesehen sind, und im Wesentlichen stegförmig ausgebildete. Führungsschienen (25) mittels einer Schraubverbindung mit in den Nuten (26) aufgenommenen Nutensteinen befestigt sind.

## Claims

1. Ceiling support to hold a medico-technical radiation source,
with a telescoping unit (2) made of a plurality of movable tubes (3) which fit inside each other,
which telescoping unit is connected via a first cable (12) with a unit for compensating the weight of the radiation source to be attached to the free end of the telescoping unit (2),
wherein the unit for compensation comprises:
a cable drum (11) for winding and unwinding the first cable (12),
a spiral winch (15) connected with the cable drum (11),
wherein the spiral winch (15) is connected via a second cable (16) with a gliding element (8) which can be displaced against the force of a pressure spring (6), and
wherein a radius of the spiral winch (15) decreases with increasing pressure force of the pressure spring (6) so that a torque compensating the weight remains essentially the same regardless of the respective length of the telescoping unit (2).

2. Ceiling support according to claim 1, wherein the spiral winch (15) is fixedly attached to the cable drum (11).

3. Ceiling support according to claim 1 or 2, wherein the decrease of the radius is not linear.

4. Ceiling support according to one of the preceding claims, wherein the spiral winch (15) is a hyperbolic spiral winch.

5. Ceiling support according to one of the preceding claims, wherein two pressure springs (6) are provided which are located on guiding tubes (5) running parallel to each other.

6. Ceiling support according to one of the preceding claims, wherein the gliding element is a crosshead (8) which can be displaced on the guiding tubes (5) against the force of the pressure springs (6).

7. Ceiling support according to one of the preceding claims, wherein the second cable (16) is guided around a roller (9) installed on the crosshead (8) and is secured on its one end to a frame (1) holding the telescoping unit (2) and on its other end at the maximum radius of the spiral winch (15).

8. Ceiling support according to one of the preceding claims, wherein the spiral winch (15) is installed between the two pressure springs (6).

9. Ceiling support according to one of the preceding claims, wherein two first cables (12) and two second cables (16) are provided.

10. Ceiling support according to one of the preceding claims, wherein the spiral winch (15) is connected with a permanent magnet brake (17) such that the spiral winch (15) is braked in case of a power failure.

11. Ceiling support according to one of the preceding claims, wherein the spiral winch (15) is connected with an electro-motor drive (20, 21, 22).

12. Ceiling support according to one of the preceding claims, wherein a device (23) is provided for setting the pre-tension of the pressure spring/springs (6) which affects the gliding element (8).

13. Ceiling support according to one of the preceding claims, wherein a device is provided for the continuous setting of the spring rate which is preferably a clamping cuff (23) which presses the pressure spring (6) against the guiding tube (5).

14. Ceiling support according to one of the preceding claims, wherein a plurality of axially running undercut grooves (26) are provided on the inner side of at least one of the tubes (3) and essentially ridge-shaped guide rails (25) are secured with a screw connection to the tenon block retained in the grooves (26).

## Revendications

1. Support plafonnier destiné à recevoir une source de rayonnement médico-technique
avec un télescope (2) formé de plusieurs longerons (3) pouvant s'emboîter l'un dans l'autre,
qui est relié par un premier câble (12) à un dispositif de compensation du poids de la source de rayonnement qui doit être monté à l'extrémité libre du télescope (2),
sachant que le dispositif de compensation comprend:
un tambour à câble (11) pour l'enroulement et le déroulement du premier câble (12), un treuil hélicoïdal (15) relié au tambour à câble (11),
sachant que le treuil hélicoïdal (15) est relié au moyen d'un deuxième câble (16) à un élément glissant (8) pouvant être déplacé contre la force d'un ressort de pression (6), et
sachant qu'un rayon du treuil hélicoïdal (15) diminue avec un effort de pression de plus en plus grand du ressort de pression (6), de sorte qu'un moment compensant le poids reste essentiellement le même indépendamment de la longueur respective du télescope (2).

2. Support plafonnier selon la revendication 1, dans lequel le treuil hélicoïdal (15) est monté de manière fixe sur le tambour à câble (11).

3. Support plafonnier selon la revendication 1 ou 2, dans lequel la diminution du rayon n'est pas linéaire.

4. Support plafonnier selon l'une quelconque des revendications précédentes, dans lequel le treuil hélicoïdal (15) est un treuil hélicoïdal hyperbolique.

5. Support plafonnier selon l'une quelconque des revendications précédentes, dans lequel sont prévus deux ressorts de pression (6) qui sont disposés sur des tubes conducteurs (5) parallèles les uns aux autres.

6. Support plafonnier selon l'une quelconque des revendications précédentes, dans lequel l'élément glissant est une traverse (8) pouvant être déplacée sur les tubes conducteurs (5) contre la force des ressorts de pression (6).

7. Support plafonnier selon l'une quelconque des revendications précédentes, dans lequel le deuxième câble (16) est guidé autour d'une bobine (9) montée sur la traverse (8), fixé à l'une de ses extrémités à un cadre (1) recevant le télescope (2) et à son autre extrémité au niveau du rayon maximal du treuil hélicoïdal (15).

8. Support plafonnier selon l'une quelconque des revendications précédentes, dans lequel le treuil hélicoïdal (15) est disposé entre les deux ressorts de pression (6).

9. Support plafonnier selon l'une quelconque des revendications précédentes, dans lequel sont prévus deux premiers câbles (12) et deux deuxièmes câbles (16).

10. Support plafonnier selon l'une quelconque des revendications précédentes, dans lequel le treuil hélicoïdal (15) est relié à un frein à aimant permanent (17), de telle manière que le treuil hélicoïdal (15) est freiné en cas de panne de courant.

11. Support plafonnier selon l'une quelconque des revendications précédentes, dans lequel le treuil hélicoïdal (15) est relié à une commande électromotrice (20, 21, 22).

12. Support plafonnier selon l'une quelconque des revendications précédentes, dans lequel est prévu un dispositif (23) pour le réglage d'une prétension du ou des ressorts de pression (6), agissant sur l'élément glissant (8).

13. Support plafonnier selon l'une quelconque des revendications précédentes, dans lequel est prévu un dispositif pour le réglage en continu des écartements du ressort, qui est de préférence une manchette de serrage (23) haubanant le ressort de pression (6) contre le tube conducteur (5).

14. Support plafonnier selon l'une quelconque des revendications précédentes, dans lequel plusieurs rainures (26) à contre-dépouille s'étendant axialement sont prévues sur un côté intérieur d'au moins un des longerons (3) et des rails de guidage (25) configurés essentiellement en forme de tige sont fixés au moyen d'un assemblage par vis aux coulisseaux logés dans les rainures (26).
